Europäisches Patentamt

European Patent Office

Office européen des brevets

(11) Publication number: **0 432 693 A1**

# EUROPEAN PATENT APPLICATION

(21) Application number: **90123694.3**

(22) Date of filing: **10.12.90**

(51) Int. Cl.⁵ **A61K 9/127**, A61K 31 685, A61K 37/02, A61K 39/00, A61K 39/385, A61K 47 48, A61K 35/14

(30) Priority: **11.12.89 JP 322217/89**
**27.04.90 JP 112424/90**
**30.05.90 JP 142391/90**
**11.09.90 JP 241591/90**

(43) Date of publication of application:
**19.06.91 Bulletin 91/25**

(84) Designated Contracting States:
**AT BE CH DE DK ES FR GB GR IT LI LU NL SE**

(71) Applicant: SUMITOMO CHEMICAL COMPANY, LIMITED
Kitahama 4-chome 5-33
Chuo-ku Osaka 541(JP)

(72) Inventor: Kumagai, Kazuo
2-12-10, Tomogaoka
Sanda-shi, Hyogo-ken(JP)
Inventor: Nabeshima, Shigeyasu
2-10-5-502, Sonehigashi-machi
Toyonaka-shi, Osaka-fu(JP)
Inventor: Ohki, Koji
1-27, Shinkawayonjo 9-chome, Kita-ku
Sapporo-shi, Hokkaido(JP)
Inventor: Ohmura, Kazutaka
3-3-14, Kitakashiwa
Kashiwa-shi, Chiba-ken(JP)
Inventor: Ikuta, Kazuyoshi
2-38, Minamijurokujonishi 9-chome, Chuo-ku
Sapporo-shi, Hokkaido(JP)
Inventor: Kato, Shiro
23-7, Fujishirodai 4-chome
Suita-shi, Osaka-fu(JP)

(74) Representative: Vossius & Partner
Siebertstrasse 4 P.O. Box 86 07 67
W-8000 München 86(DE)

(54) Liposomes cytotoxic to virus-infected cells.

(57) Liposomes cytotoxic to virus-infected cells which comprises as membrane constituents a phosphatidyl-choline having phase-transition temperature of below 37°C, an acidic phospholipid, and cholesterol. Similar liposomes which additionally comprise a phospholipid linked to a peptide possessing anti-HIV activity. said peptide comprising an amino acid sequence:
Asn-Phe-Pro-Leu-Ile-Ile-Lys-Asn-Leu-Lys-Ile-
Glu-Asp-Ser-Asp-Thr-Tyr-Ile-Cys-Glu-Val-Glu-
Asp-Gln-Lys-Glu-Glu
wherein the Cys residue is L-cysteine, or S-benzylated or S-acetamidomethylated derivative of L-cysteine.

## LIPOSOMES CYTOTOXIC TO VIRUS-INFECTED CELLS

The present invention relates to a therapeutic or prophylactic agent for viral infections. More particularly, it relates to novel liposomes selectively cytotoxic to virus-infected cells.

Various therapeutic or prophylactic agents have been developed for treating various diseases caused by virus infections. However, only a few agents are known to be effective and practically useful. So far nucleic acid derivatives such as aciclovir and adenine arabinoside are sole agents known to be useful as therapeutic agents for viral (herpes simplex virus) infection. Accordingly, there is a need to develop other effective therapeutic agents for treatment of viral infections.

AIDS, one of typical diseases caused by virus infections, is an immunodeficiency disease caused by human immunodeficiency virus (HIV). Thus far, azidothymidine (AZT) is a sole therapeutic agent practically available for treating AIDS. AZT is a nucleoside analogue which exhibits anti-HIV activity by inhibiting the reverse transcription of HIV-RNA which produces corresponding DNA (Mitsuya, H. et al., Proc. Natl. Acad. Sci. USA. 84, 2033 -2037, 1987). Although AZT may inhibit HIV-infection on host cells, it cannot inhibit HIV replication once persistent infection on host cells has been established and the cells have acquired HIV-genome in the form of provirus (Nakashima, H. et al., Antimicrob. Agents Chemother., 30, 933-937, 1986). AZT has an additional drawback that it possesses an extensive side-effect on bone marrow, i e., hematopoietic organ. Thus, AZT is not a satisfactory agent for treatment of AIDS although it has anti-HIV activity and its clinical effect has been recognized. Accordingly, for more effective and safer treatment of AIDS, it is necessary to selectively kill persistently-infected cells which replicate HIV, and eventually inhibit the replication of HIV.

On the other hand, Epstein-Barr virus (EBV), a herpes virus, is an oncogenic virus which infects human B lymphocytes and causes infectious mononucleosis, Burkitt's lymphoma and epipharynx cancer. There is no established anti-virus agent for the EBV infections although anti-cancer drugs such as cyclophosphamide have been used for the treatment of the cancer. EBV has carcinogenic property and causes canceration of infected cells. Accordingly, for more effective treatment of EBV infections, it appears necessary to selectively kill EBV-infected cells.

A liposome preparation in which diphtheria toxin fragment A has been entrapped is known to selectively kill virus-infected cells; see JP-A-252425/1985 and 290824/1988 . On the other hand, it is reported that a CD4-ricin A chain conjugate which has been prepared by linking ricin A chain to a soluble CD4 molecule (Till, M. A. et al., Science, 242, 1166-1168, 1988), and a recombinant fused protein comprising CD4 and Pseudomonas exotoxin A (Chaudhary, V. K. et al., Nature, 335, 369-372, 1988) have successfully killed HIV-infected cells in vitro. Further, immunotoxin comprising an anti-gp120 monoclonal antibody linked to ricin A chain has been shown to be effective for killing of HIV-infected cells (Matsushita, S. et al., Anti-gp120 immunotoxin against HIV infected cells, Fourth International Conference on AIDS, Stockholm, Book 1, p234, 1988). CD4 is a differentiation antigen of human T lymphocytes and has an affinity to the HIV envelope glycoprotein, gp120. It is known that gp120 is expressed on the surface of HIV-infected cells. Accordingly, the above-mentioned preparations which comprise CD4 or anti-gp120 monoclonal antibody linked to a toxin or its fragment appear to selectively bind to and kill the HIV-infected cells.

As noted above, several agents and methods for selective killing of HIV-infected cells are already known. However, they are not satisfactory as therapeutic agents in respect of safety and stability.

It is the object of the invention to provide an effective agent for treatment of viral infections. This object could be achieved on the basis of the surprising finding that a liposome comprising phosphatidylcholine having phase-transition temperature of below 37° C, acidic phospholipid, and cholesterol has an ability to selectively kill virus-infected cells, and that the liposomes are quite useful as a therapeutic agent for the treatment of viral infections.

In addition, it has also been found that a liposome which is similar to the just-mentioned liposome but additionally contains a peptide consisting of 68 - 94 amino acid sequence of human CD4 and capable of showing anti-HIV activity, or its derivative, exerts highly selective killing activity against HIV-infected cells, and that the liposomes are specifically useful as a therapeutic agent for the treatment of AIDS.

Thus, the present invention provides liposomes cytotoxic to virus-infected cells which comprise as membrane constituents a phosphatidylcholine having a phase-transition temperature of below 37° C, an acidic phospholipid, and cholesterol. The liposomes of this type may be refferred to as "liposomes of Type A" hereinafter.

The present invention also provides liposomes cytotoxic to HIV-infected cells which comprise as membrane constituents:

(a) phospholipid linked to a peptide possessing anti-HIV activity, said peptide comprising an amino acid sequence:

Asn-Phe-Pro-Leu-Ile-Ile-Lys-Asn-Leu-Lys-Ile-
Glu-Asp-Ser-Asp-Thr-Tyr-Ile-Cys-Glu-Val-Glu-
Asp-Gln-Lys-Glu-Glu

wherein the Cys residue is L-cysteine, or S-benzylated or S-acetamidomethylated derivative of L-cysteine;

(b) phosphatidylcholine having phase-transition temperature of below 37° C;

(c) acidic phospholipid; and

(d) cholesterol.

The liposomes of this type may be referred to as "liposomes of Type B" hereinafter.

The invention also provides for the use of the liposomes mentioned above for preparing a medicament for treating virus infections, including HIV- and EBV-infections.

In the accompanying drawings:

Fig. 1 shows growth inhibitory action of the liposomes of the invention prepared in Example 1 on HIV-infected cells (●-●) and uninfected cells (O-O).

Fig. 2 shows growth inhibitory action of the liposomes of the invention prepared in Example 2 on HIV-infected cells (●-●) and uninfected cells (O-O).

Fig. 3 shows growth inhibitory action of the liposomes of the invention prepared in Example 3 on HIV-infected cells (●-●) and uninfected cells (O-O).

Fig. 4 shows growth inhibitory action of the liposomes of the invention prepared in Example 4 on HIV-infected cells (●-●) and uninfected cells (O-O).

Fig. 5 shows growth inhibitory action of the liposomes of the invention prepared in Example 5 on EBV-infected cells, Raji (●-●) and p3HR-1 (▲-▲), and uninfected cells (O-O).

Fig. 6 shows growth inhibitory action of the liposomes of the invention prepared in Example 6 on EBV-infected cells, Raji (●-●) and p3HR-1 (▲-▲), and uninfected cells (O-O).

Fig. 7 shows growth inhibitory action of the liposomes of the invention prepared in Comparative Example 1 on HIV-infected cells (●-●) and uninfected cells (O-O).

Fig. 8 shows growth inhibitory action of the liposomes of the invention prepared in Comparative Example 2 on HIV-infected cells (●-●) and uninfected cells (O-O).

Fig. 9 shows growth inhibitory action of the liposomes of the invention prepared in Example 13 on HIV-infected cells (●-●) and uninfected cells (O-O).

Fig. 10 shows growth inhibitory action of the liposomes of the invention prepared in Example 14 on HIV-infected cells (●-●) and uninfected cells (O-O).

Fig. 11 shows growth inhibitory action of the liposomes of the invention prepared in Example 15 on HIV-infected cells (●-●) and uninfected cells (O-O).

Fig. 12 shows growth inhibitory action of the liposomes of the invention prepared in Comparative Example 3 on HIV-infected cells (●-●) and uninfected cells (O-O).

In each of the figures, the ordinate represents a percentage of growth inhibition (%) of the cells and the abscissa represents $OD_{540}$ of liposomes.

In the following description, the term "phase-transition temperature" means gel to liquid-crystalline phase transition temperature. Bilayered membrane of phospholipid exists in the form of gel phase turning to a hard membrane at temperature below the phase-transition temperature and exists in the form of liquid-crystalline phase turning to a soft membrane at temperature above the phase-transition temperature. The phase-transition temperature varies depending on the nature of fatty acids contained in the phospholipid.

Liposomes are closed vesicles consisting of lipid bilayered membrane and liquid entrapped therein. It is well known that such lipid bilayered structure is very similar to biomembrane. Since liposomes are usually prepared using natural materials found in cell membranes, they are remarkably bio-compatible and show minimum toxicity and antigenicity, if any. Liposomes are classified into two groups from morphological view point, i.e., multilamellar vesicle (MLV) and unilamellar vesicle (ULV), the latter being further subclassified into large unilamellar vesicle (LUV) and small unilamellar vesicle (SUV). The liposomes of the present invention may be any form of them.

Examples of phosphatidylcholines having phase-transition temperature of below 37° C used in the present invention are lecithin of animal origin, such as egg yolk lecithin, soybean lecithin, and phosphatidylcholines in which the acyl group has been substituted by lauroyl, myristoyl, oleoyl or the like group. Most preferred are an egg yolk lecithin having phase-transition temperature of -15 ~-7° C, dilauroylphosphatidylcholine (DLPC) having phase-transition temperature of $0\,°C$, and dimyristoylphosphatidylcholine (DMPC) having phase-transition temperature of $23\,°C$.

Specific examples of acidic phospholipids are phosphatidylserine, phosphatidylglycerol, phosphatidylinositol, and phosphatidic acid of animal origin, and the just-mentioned materials in which the acyl group contained therein has been substituted by lauroyl, myristoyl, palmitoyl, stearoyl, oleoyl, or the like group. Preferred acidic phospholipid is phosphatidylserine and phosphatidylglycerol of animal origin. Anti-oxidants such as 2-tocopherol may be added thereto, if desired.

Molar ratio of phosphatidylcholine having phase-transition temperature of below $37\,°C$, acidic phospholipid, and cholesterol in the liposomes of the present invention is preferably 5:1:2. However, the molar ratio may be changed as far as the selectivity to virus-infected cells is retained. Thus, the molar percentages of the phosphatidylcholine, the acidic phospholipid, and the cholesterol may be 60-85%, 5-20%, and 10-35%, respectively.

In the liposomes of Type B, the phospholipid linked to the peptide is added to the liposomes of Type A in the molar ratio of 0.1-10%. In other words, the molar percentage of the phospholipid, the phosphatidylcholine, the acidic phospholipid, and the cholesterol in the liposomes of Type B may be 0.1-10%, 55-85%, 5-20%, and 10-35%, respectively.

The number of the peptides to be linked to the individual liposomes of Type B is preferably in the range of 40-120, although up to about 8000 peptides can theoretically be linked to a single liposome.

Human CD4 is a glycoprotein having a molecular weight of about 55 kilodalton and its primary structure is known (Maddon, P. J. et al., Cell, 42, 93-104, 1985). Extensive study has been done in order to determine a domain of CD4 responsible for interaction with HIV using synthesized peptides, and has suggested that the domain was constituted by twenty-seven (27) amino acids ranging from the amino acid at position 68 to the amino acid at position 94, starting from N-terminal, said amino acid sequence being Asn-Phe-Pro-Leu-Ile-Ile-Lys-Asn-Leu-Lys-Ile-Glu-Asp-ser-Asp-Thr-Tyr-Ile-Cys-Glu-Val-Glu-Asp-Gln-Lys-Glu-Glu (Hayashi, Y. et al., Arch. Virol., 105, 129-135, 1989; Ohki, K. et al., Abstract Book for 3rd Research Conference on AIDS (at Matsue), p79, 1989; and Ohki, K. et al., Abstract Book for 37th General Meeting of The Society of Japanese Virologists (at Osaka), p83, 1989). Further, a peptide compound containing the same amino acid sequence as mentioned above (referred to as CD4(68-94) hereinafter) has been shown to inhibit HIV-infection (JP-A-172053/1989). In addition, it has also been reported that modified CD4(68-94), such as CD4(68-94)Bzl in which the cysteine residue has been benzylated, and CD4(68-94)Acm in which the cysteine residue has been modified with acetamidomethyl group, exert higher inhibitory activity against HIV-infection (JP-A-172053/1989). In the present invention, the use of CD4(68-94), CD4(68-94)Bzl, and CD4(68-94)Acm is most preferred.

The peptides mentioned above, CD4(68-94), CD4(68-94)Bzl, and CD4(68-94)Acm, are obtainable by the method disclosed in JP-A-172053/1989. The method includes synthesis of fragment peptides of CD4 molecule on the basis of the amino acid sequence deduced from the DNA sequence encoding the CD4 molecule (Maddon, P. J. et al., Cell, 42, 93-104, 1985). For the synthesis of the fragment peptides, the use of solid phase synthesis using Fmoc (9-fluorenylmethyloxycarbonyl) amino acid is preferred (Sheppard, R. C. et al., J. Chem. Soc., Chem. Commun., 165-166, 1985). In this method, pentafluorophenyl (Pfp) Fmoc amino acid ester corresponding to the C-terminal amino acid of each peptides is first linked to a p-alkoxybenzyl alcohol resin in the presence of 4-dimethylaminopyridine and dimethylformamide (DMF). Successive amino acids in the form of Fmoc-amino acid Pfp ester are then linked one by one to the amino acid already linked to the resin by coupling reaction. After completion of all of the coupling reactions, the resin linked to the resultant peptide is treated with 20% piperidine in DMF to remove N-terminal Fmoc group. Additional treatment of the resin with trifluoroacetic acid (TFA)/ thioanisole in the presence of m-cresol at room temperature provides the desired peptide which has been deprotected.

Alternative method for preparing the peptides employs t-butyloxycarbonyl (Boc)-amino acids on the solid phase synthesis (Merrifield, R. B., J. Am. Chem. Soc., 85, 2149-2154, 1963).

In both methods mentioned above, modification of amino acids, if any, should be conducted so that modified amino acids may remain stable under the deprotection conditions.

Other synthetic methods may be available for preparing the peptides. In addition, the peptides may be prepared by recombinant DNA technology in which DNA corresponding to the peptides is obtained in the first place, then the DNA is introduced into an appropriate vector, the vector is incorporated into animal cells or microorganisms, and the cells are cultivated. The resultant peptides may be chemically modified, if desired.

Linkage of the peptides to liposomes can be accomplished according to any of conventional methods which are known to be useful for preparing liposomes linked to antibodies (Methods in Enzymology, 149, 111-119, 1987). According to the known method, a peptide-phospholipid conjugate is first prepared by reaction of a thiol group-introduced peptide with phospholipid having a group reactive with the thiol group. Then phosphatidylcholine having phase-transition temperature of below 37°C, acidic phospholipid, and cholesterol, which are all additional components required for preparing the liposomes of the invention, are then added to the conjugate to obtain liposomes. Alternative method comprises first preparing liposomes with phospholipid having a group reactive with a thiol group, phosphatidylcholine having phase-transition temperature of below 37°C, acidic phospholipid, and cholesterol, and then reacting the resultant membrane with the thiol group-introduced peptide to prepare the liposomes of the invention.

In the above methods, the introduction of a thiol group into peptide can be attained by combining a bifunctional protein modificator such as N-succinimidyl pyridyldithiopropionate (SPDP) to the peptide, followed by reduction of the resultant product. As for CD4(68-94) peptide, the thiol group already existing in the cysteine residue of the peptide may be used.

Examples of the phospholipid having a group reactive with a thiol group are pyridyldithiopropionylphosphatidylethanolamine (PDP-PE) and 4-(p-maleimidophenyl)-butyrylphosphatidylethanolamine (MPB-PE), which are prepared by reaction of phosphatidylethanolamine with SPDP and N-succinimidyl 4-(p-maleimidophenyl)butyrate, respectively. The phosphatidylethanolamine used in the above reaction may be of animal origin or the one in which the acyl group contained therein has been substituted by lauroyl, myristoyl, palmitoyl, stearoyl, oleoyl group, etc. The former is most preferred.

It is known that interchange and transfer of membrane constituents occur when liposomes come in contact with living cells (Mashino, K. et al., J. Biochem., 94, 821 - 831, 1983; and Hestis, W. H. et al., J. Biol. Chem., 261, 16274 - 16278, 1986). On the other hand, it is also known that the cell membrane of persistently virus-infected cells has a different composition as compared with that before the infection. According to the recent report, the envelope glycoproteins of HIV (gp120, gp41) are expressed on the surface of HIV-infected cells, and also there is a change in lipid metabolism in HIV-infected cells such that synthesis of phospholipids decreases and synthesis of triglyceride and cholesterol esters increases (Lynn, W. S. et al., Virology, 163, 43 - 51, 1988). Accordingly, it is believed that the mechanism by which the liposomes of the present invention selectively kill virus-infected cells and give no harm to uninfected normal cells is that the liposomes only contact with the infected cells having altered membrane composition, such contact causing interchange and transfer of membrane constituents which result in serious damage to the cells. The liposomes of type B, which are linked to a peptide comprising 68 - 94 amino acid sequence of human CD4 and possessing anti-HIV activity, or chemically-modified analogue of it are also capable of selectively contacting HIV-infected cells, and causing great damage to the infected-cells via interchange and/or transfer of membrane constituents.

The liposomes of the invention can be prepared by any one of conventional methods such as listed below.

(1) Components for liposome membrane are mixed together in an appropriate solvent, and the solvent is removed by evaporation to yield a thin lipid film, which is then sufficiently hydrated by addition of a buffer solution, to give liposomes of MLV type.

(2) Liposomes of MLV type are filtered through a membrane filter to provide liposomes having uniform particle size.

(3) Liposomes of MLV type are treated with ultrasonic wave or high pressure blast emulsifier to give liposomes of SUV type.

The buffer solution used in the above processes may be selected from various buffer solutions known as useful for the production of liposomes, such as phosphate buffers, citrate buffers, lactate buffers, acetate buffers, and the like. Preferred pH of the buffer solutions is in the range of 6 - 8, and preferred concentration of the solutions is in the range of 5 - 50 mM. Buffer solutions which have been made isotonic by the addition of sodium chloride, glucose or sucrose are also useful. Further, physiological saline may be employed in place of buffer solutions.

The liposomes of the present invention may entrap a drug. The drug may be a toxin capable of inhibiting protein synthesis in cells, such as diphtheria toxin fragment A, or an anti-virus agent.

Since the liposomes of the present invention are capable of selectively killing virus-infected cells, they are useful as a therapeutic agent or a prophylactic agent for viral infections, particularly for AIDS. The liposomes of the invention are physiologically safe because they consist of only lipid components or lipid components plus CD4 fragment peptide having the same amino acid sequence as of human CD4. The liposomes may be administered intravenously to patients suffering from AIDS or AIDS-related syndrome (ARC), or asymptomatic carriers, via injection or drip infusion. Intravenous administration of the liposomes of the invention may be of advantage because HIV-infected cells are found mainly in blood.

As stated above, the liposomes of the present invention selectively kill HIV-infected cells. Accordingly, the liposomes are very useful for therapeutic or prophylactic treatment of viral infections. It should be also emphasized that the liposomes of the invention may be administered for a long period of time without fear of side-effects because they show no toxicity to uninfected normal cells.

The following detailed examples are presented by way of illustration of certain specific embodiments of the invention and should not be construed as limiting in any respect.

Example 1

Dimyristoylphosphatidylcholine (DMPC) having phase-transition temperature of 23°C (Nippon Oil and Fats)(53.8 mg), phosphatidylserine (PS) from bovine brain (Sigma)(12.2 mg), and cholesterol (CH)(Wako Pure Chemical Industries)(12.2 mg) were placed in a pear-shaped flask (molar ratio of three material was 5:1:2). A mixture of chloroform and methanol (9:1 v/v)(5 ml) was added thereto and mixed well until a solution was obtained. The organic solvent was removed under reduced pressure using a rotary evaporator, thereby thin film of lipids was formed on the inner wall of the flask. To the flask was added 10 mM phosphate buffer (5 ml), pH 7.2, containing 300 mM sucrose, and the air in the flask was replaced by nitrogen gas. The flask was kept at 30°C for one hour to proceed sufficient hydration, and the content was stirred for 10 minutes using a vortex mixer to yield a white suspension. The suspension was passed through a polycarbonate membrane filter having a pore diameter of 1 $\mu$m (Nuclepore) ten times, and then through a polycarbonate membrane filter having a pore diameter of 200 nm (Nuclepore) ten times. This procedure yielded liposomes composed of DMPC-PS-CH (molar ratio 5:1:2) having uniform size (Average vesicle size was 178 nm according to light-scattering method). Turbidity at 540 nM ($OD_{540}$) of the liposomes thus obtained was 1.71.

Cytotoxicity of the liposomes thus obtained on virus-infected cells was determined in vitro. For this purpose, persistently HIV-infected human cells (MOLT-4/ HTLV-III$_B$)(2.5 x $10^6$ cells) were suspended in 0.67 ml of RPMI-1640 medium containing 10% fetal bovine serum. The suspension was admixed with a liposome solution (0.33 ml) which had been adjusted to $OD_{540}$ 0.1 - 1.5 by dilution with 10 mM phosphate buffer, pH 7.2, containing 300 mM sucrose. The mixture was gently shaken for 1 hour at 37°C to allow the liposomes to contact the cells. RPMI-1640 medium containing 10% fetal bovine serum (4 ml) was then added, and the mixture was incubated for 3 days in a $CO_2$ gas incubator (37°C, 5% $CO_2$). After the incubation, the number of viable cells were counted by the trypan blue dye exclusion method. Control test was conducted in the same way by the use of phosphate-buffered saline (PBS) in place of the liposome solution. Growth inhibition (%) was determined according to the following equation.

$$\text{Growth Inhibition (\%)} = \left(1 - \frac{\text{Number of Viable Cells after Treatment with Liposome Solution}}{\text{Number of Viable Cells in Control}}\right) \times 100$$

In order to investigate the selectivity of the liposomes of the invention, the same experiment as above was conducted using uninfected MOLT-4 cells.

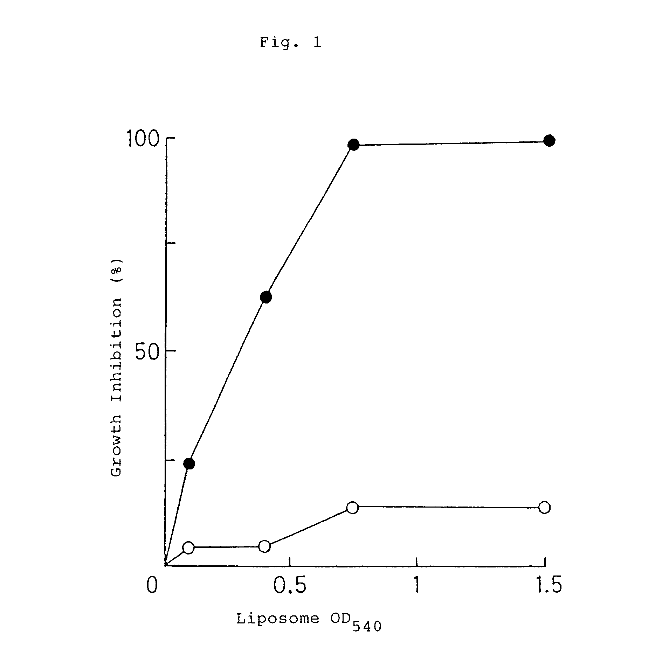

The results are shown in Fig. 1. In the figure, ●-● and O-O respectively show growth inhibition on HIV-infected cells (MOLT-4/ HTLV-III$_B$) and uninfected cells (MOLT-4). As shown in Fig. 1, the liposomes of the invention showed strong growth inhibition on HIV-infected cells. The liposomes completely inhibited the growth of the infected cells when $OD_{540}$ was larger than 0.75. On the other hand, the liposomes did not show any inhibitory action on the growth of the uninfected cells even at the highest concentration of the liposomes. Accordingly, it was concluded that the liposomes of the invention have selective cytotoxicity to virus-infected cells.

Example 2

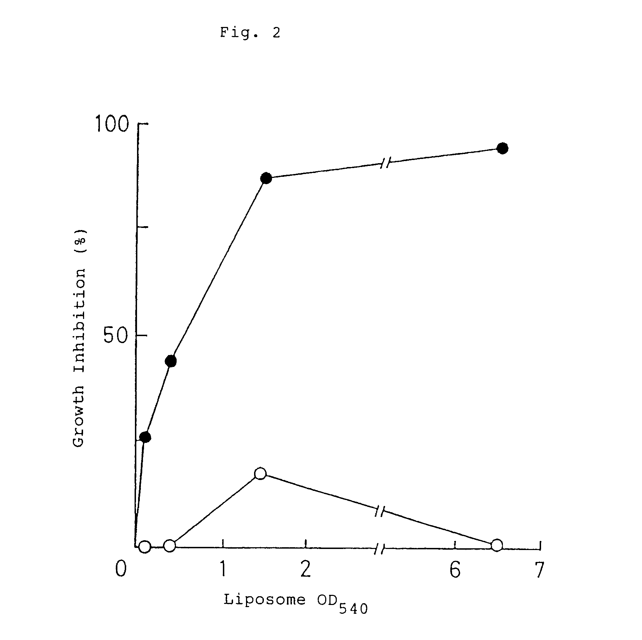

The procedure described in Example 1 was repeated except that the suspension was once passed through a membrane filter having a pore diameter of 1.2 $\mu$m (Sartorius) to prepare liposomes of MLV type. $OD_{540}$ of thus obtained liposomes was 7.65. Cytotoxicity of the liposomes was determined according to the method described in Example 1. The results are shown in Fig. 2. The liposomes of the invention of MLV type showed an intense growth-inhibitory action to HIV-infected cells in the wide concentration range of the liposomes. More than 90% growth inhibition was obtained when $OD_{540}$ was larger than 1.5. On the other hand, the liposomes showed little inhibitory action on the growth of the uninfected cells. This test revealed that the liposomes of the present invention have selective cytotoxicity to virus-infected cells even in the form of MLV.

Example 3

The procedure described in Example 1 was repeated except that dilauroylphosphatidylcholine having phase-transition temperature of $0°C$ (DLPC, Nippon Oil and Fats)(49.3 mg) was used in place of DMPC to prepare liposomes composed of DLPC-PS-CH (molar ratio 5:1:2). $OD_{540}$ of the resultant liposomes was 1.64.

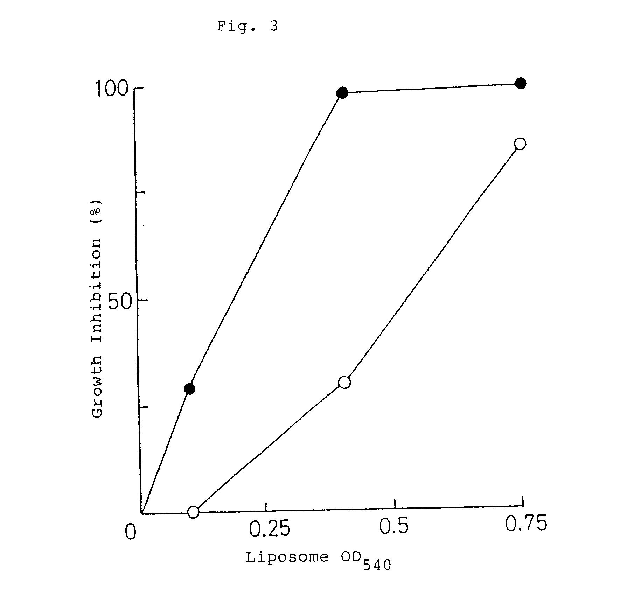

Cytotoxicity of the liposomes was determined according to the method described in Example 1. The results are shown in Fig. 3. The liposomes completely inhibited the growth of the infected cells when $OD_{540}$ was larger than 0.4. This indicates that the liposomes composed of DLPC-PS-CH (molar ratio 5:1:2) have stronger cytotoxicity than the liposomes composed of DMPC-PS-CH (molar ratio 5:1:2) obtained in Examples 1 and 2. On the other hand, the liposomes of this Example did not inhibit the growth of the uninfected cells when $OD_{540}$ was below 0.4, although they showed the inhibitory action at a concentration of above 0.4. Thus, the liposomes composed of DLPC-PS-CH (molar ratio 5:1:2) have selective cytotoxicity to virus-infected cells.

Example 4

The procedure described in Example 1 was repeated except that egg yolk lecithin (egg phosphatidylcholine) having phase-transition temperature of $-15°C \sim -7°C$ (PC, Nippon Oil and Fats)(61.0 mg) was used in place of DMPC to prepare liposomes composed of PC-PS-CH (molar ratio 5:1:2). $OD_{540}$ of the resultant liposomes was 3.12.

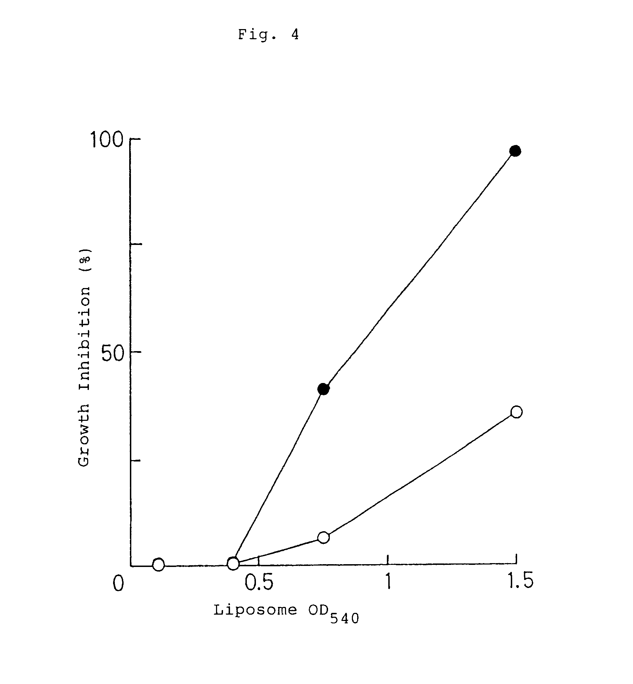

Cytotoxicity of the liposomes was determined according to the method described in Example 1. The results are shown in Fig. 4. The liposomes showed cytotoxicity on HIV-infected cells when $OD_{540}$ was larger than 0.75. On the other hand, the liposomes hardly exhibited inhibitory action on the growth of the uninfected cells. Thus, the liposomes composed of PC-PS-CH (molar ratio 5:1:2) have selective cytotoxicity to virus-infected cells.

Example 5

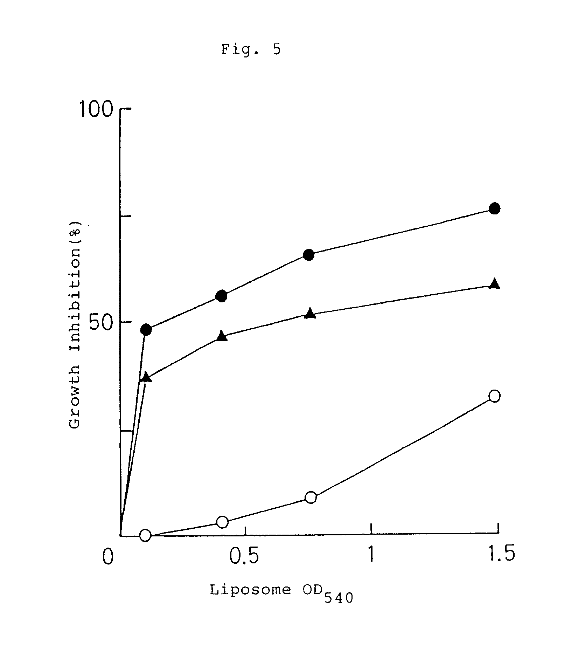

Liposomes composed of DMPC-PS-CH (molar ratio 5:1:2) were prepared according to the method described in Example 1. $OD_{540}$ of the resultant liposomes was 1.68. Cytotoxicity of the liposomes to Epstein-Barr virus (EBV)-infected cells was determined by the method described in Example 1, using human cell lines Raji and P3HR-1 derived from Burkitt lymphoma as EBV-infected cells. The results are shown in Fig. 5. The liposomes exhibited intense growth-inhibitory action on both Raji (●-●) and p3HR-1 (▲-▲) dose-dependently. On the other hand, the liposomes did not show inhibitory action on uninfected cells (MOLT-4) (O-O). Accordingly, it was concluded that the liposomes of the invention also have selective cytotoxicity to EBV-infected cells.

Example 6

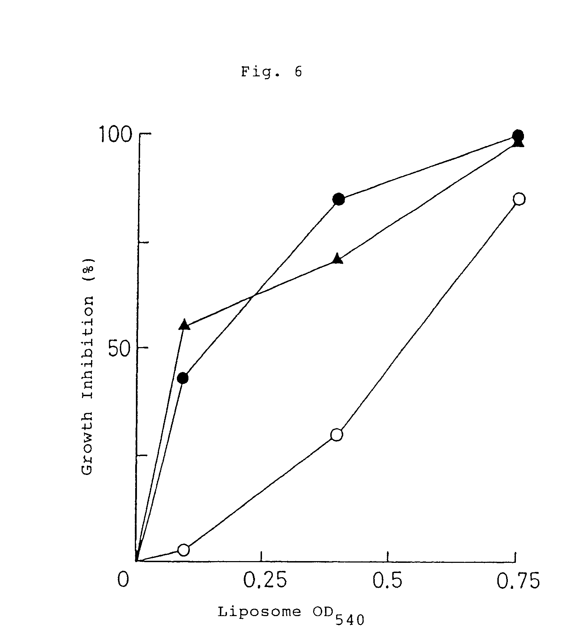

Liposomes composed of DLPC-PS-CH (molar ratio 5:1:2) were prepared according to the method described in Example 3. $OD_{540}$ of the resultant liposomes was 1.45. Cytotoxicity of the liposomes to EBV-infected cells was determined according to the method described in Example 5. The results are shown in Fig. 6. The liposomes showed intense growth-inhibitory action on both Raji (●-●) and p3HR-1 (▲-▲) when $OD_{540}$ was larger than 0.1. On the other hand, the liposomes did not show significant cytotoxicity on the uninfected cells (O-O) at a concentration of below 0.4. It was therefore concluded that the liposomes of the invention obtained in this Example also have selective cytotoxicity to EBV-infected cells.

Comparative Example 1

The procedure of Example 1 was repeated except that dipalmitoylphosphatidylcholine having phase-transition temperature of $42°C$ (DPPC, Nippon Oil and Fats)(58.2 mg) was used in place of DMPC and that the hydration was conducted at $47°C$ rather than $30°C$, to prepare liposomes composed of DPPC-PS-CH (molar ratio 5:1:2). $OD_{540}$ of the liposomes was 2.16.

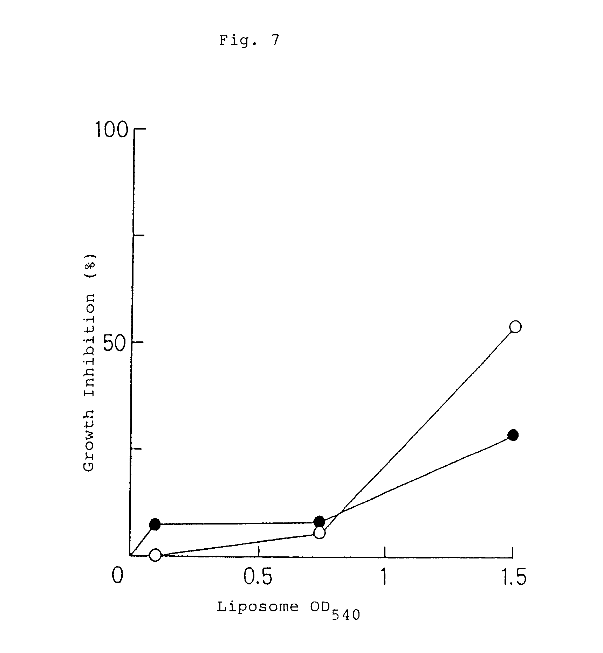

Cytotoxicity of the liposomes to virus-infected cells was determined according to the method described in Example 1. The results are shown in Fig. 7. The liposomes hardly showed inhibitory action on the growth of HIV-infected cells. They showed no selectivity to the infected cells either. This indicates that liposomes comprising as a major component DPPC having phase-transition temperature of above 37°C, particularly 42°C, have no selective cytotoxicity on virus-infected cells.

Comparative Example 2

The procedure of Example 1 was repeated except that distearoylphosphatidylcholine having phase-transition temperature of 55°C (DSPC, Nippon Oil and Fats)(62.6 mg) was used instead of DMPC and that the hydration was conducted at 60°C instead of 30°C, to prepare liposomes composed of DSPC-PS-CH (molar ratio 5:1:2). $OD_{540}$ of the liposomes was 2.85.

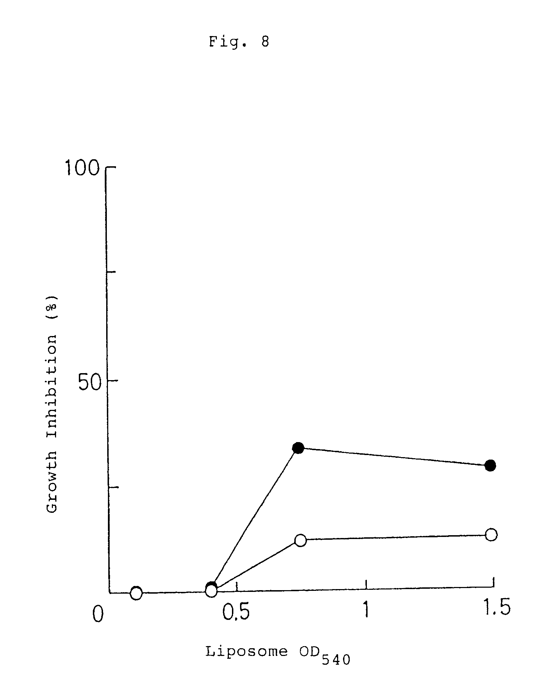

Cytotoxicity of the liposomes to virus-infected cells was determined according to the method described in Example 1. The results are shown in Fig. 8. The maximum percentage of growth inhibition of HIV-infected cells by the liposomes was only 34% and they did not exert selectivity to the infected cells. These results indicate that liposomes comprising as a major component DSPC having phase-transition temperature of above 37°C, particularly 55°C, have no selective cytotoxicity to virus-infected cells.

Example 7 Preparation of CD4(68-94) peptide

Fmoc-Glu (OBzl)-O-pentafluorophenyl ($Pf_p$) ester (1 mmol) was linked via an ester bond to p-alkoxybenzyl alcohol resin (0.2 mmol)(0.35 meq OH/g, Polystyrene- 1% Divinylbenzene Copolymer, Kokusan Chemical Works) by the use of 4-dimethylaminopyridine (DMAP)(0.2 mmol) as a catalyst and dimethylformamide (DMF) as a solvent.

Side chains protection of Fmoc-amino acid derivatives (MilliGen/Biosearch) was Asp (OBu$^t$), Glu (OBu$^t$), Thr (Bu$^t$), Ser (Bu$^t$), Tyr (Bu$^t$), Lys (Boc), and Cys (Acm). Coupling reactions for peptide chain elongation were all conducted using Pfp active ester (2.5 eq) in the presence of 1-hydroxybenzotriazole (HOBT)(0.2 mmol) as a catalyst in DMF. Removal of respective Fmoc groups was conducted by the use of 20% piperidine in DMF. All of the coupling reactions were monitored using ninhydrin.

After completion of all coupling reactions, a Fmoc group remained at N-terminal of resultant peptide was removed using 20% piperidine/ DMF to make the N-terminal amino acid free. The peptide was then treated with TFA-thioanisole in the presence of m-cresole for 3 hours at room temperature for the purpose of removing all protecting groups and separating the peptide from the resin. The reaction mixture was filtered through a glass filter to remove the resin. The filtrate was concentrated at room temperature and added with ethyl ether to yield powders. The powders were dissolved in an appropriate buffer solution and the resultant solution was subjected to gel-filtration using Sephadex G-25 (Pharmacia). Elution with 0.5N acetic acid gave desired peptide.

The thus obtained peptide was further purified by reversed phase HPLC [Column: Nucleosil 100-5C18 (4.0 x 150 mm)(M. Nergel); Solvent: 10 - 60% acetonitrile in 0.1% TFA (gradient); Flow rate: 1 ml/ minute; Detection: UV at 210, 260 nm]. CD4(68-94) peptide was recovered at retention time of 16.8 minutes. Amino acid analysis of resultant CD4(68-94) peptide revealed that the product had the same amino acid composition as theoretically calculated.

Example 8 Preparation of CD4(68-94)Bzl peptide

Boc-Glu (OBzl)-phenylacetamidomethyl resin (0.5 mmol scale, Applied Biosystems) was used as a starting material for C-terminal amino acid, glutamic acid. Stepwise elongation of peptide chain on the resin was conducted using a Applied Biosystems Model 430A peptide synthesizer (Program version 1.40). Side chain protection of Boc amino acid derivatives (Peptide Institute) used in the elongation of peptide chain was Asp (OcHex), Glu (OcHex), Thr (Bzl), Ser (Bzl), Tyr (Br-Z), Lys (Cl-Z), and Cys (Bzl). Every coupling reaction was conducted in DMF after synthesis of corresponding symmetric acid anhydride of Boc-amino acid (2.0 eq.) using dicyclohexylcarbodiimide (DCC) in a mixture of dichloromethane and DMF. For removal of Boc groups, 50% TFA-dichloromethane solution was employed.

After completion of all coupling reactions, the resultant peptide was treated with 10% anisole/ anhydrous hydrogen fluoride (HF) at -5°C for 30 minutes to remove Boc group at N-terminal and all protection groups excepting Bzl on cysteine residue and to separate the peptide from the resin. HF was evaporated under reduced pressure and the residue was washed with ethyl ether. The peptide was dissolved in TFA and the solution was filtered through a glass filter to remove the resin. The filtrate was condensed under reduced pressure, and the residue was added with ethyl ether to give powders. The crude peptide thus obtained was purified using reversed phase HPLC as described in Example 7.

Example 9 Preparation of CD4(68-94)Acm peptide

The procedure described in Example 8 was repeated except that Cys (Acm) was used in place of Cys (Bzl) to provide CD4(68-94)Acm peptide.

Example 10 Introduction of thiol group into CD4(68-94) peptide

CD4(68-94) peptide (8 mg) was dissolved in 50 mM phosphate buffer (2 ml), pH 7. To the solution, 60 mM SPDP (172 $\mu$l) dissolved in ethanol was added, and the mixture was gently stirred at room temperature for 2 hours in a sealed vessel. Dithiothreitol (16.7 mg) was then added to the reaction mixture, and the mixture was gently stirred again at room temperature for 1.5 hours in a sealed vessel. The resultant reaction mixture was applied to a column of Sephadex G-15 (16 ml)(Pharmacia) equilibrated with 50 mM phosphate buffer, pH 7, and eluted with the same buffer. Eluates were analyzed by reversed phase HPLC [Column: YMC-Pack AM-303 (5$\mu$, 100Å), 4.6 x 250 mm (YMC); Eluent, A solution: water containing 1% TFA; B solution: acetonitrile containing 0.1% TFA; Gradient: 0% B→70% B in 35 minutes; Flow rate: 1 ml minute; Temperature: room temperature; Detection: UV at 210 nm]. The retention time of CD4(68-94) peptide into which thiol group had been introduced was 28.0 minutes, while that of unreacted CD4(68-94) peptide was 23.6 minutes. 7.2 mg of purified thiol-product was thus obtained. Content of the thiol groups in the product was determined by a conventional method using 2,2'-dithiopyridine (Grassetti, D.R. & Murray, J.F.Jr., Arch. Biochem. Biophys. 119, 41-49. 1967), which revealed that one molecule of the peptide contained three thiol groups.

Example 11 Introduction of thiol group into CD4(68-94)Bzl peptide

The procedure described in Example 10 was repeated except that CD4(68-94)Bzl peptide was used in place of CD4(68-94) peptide to obtain CD4(68-94)Bzl peptide into which 2.0 thiol groups per molecule had been introduced. Yield was 7.0 mg.

Example 12 Introduction of thiol group into CD4(68-94)Acm peptide

The procedure described in Example 10 was repeated except that CD4(68-94)Acm peptide was used in place of CD4(68-94) peptide to obtain CD4(68-94)Acm peptide into which 2.1 thiol groups per molecule had been introduced. Yield was 7.1 mg.

Example 13

Pyridyldithiopropionylphosphatidylethanolamine (PDP-PE)(46.2 mg) was prepared using phosphatidylethanolamine (PE, Sigma) (50 mg) from bovine brain and N-succinimidyl pyridyldithiopropionate (SPDP, Pierce)(31.3 mg) according to the method of Martin, F.J. et al., [Biochemistry, 20, 4229-4238, 1981]. The resultant PDP-PE (1.9 mg) was placed in a vial and added with 50 mM phosphate buffer (1 ml), pH 7.5, containing the thiol group-introduced CD4(68-94) peptide (0.9 mg) prepared in Example 10 and then acetonitrile (1 ml) to make a solution. After replacement of the air in the vial with nitrogen gas, the vial was sealed, and the content was gently stirred at 4°C for 16 hours to allow the peptide to conjugate to the phospholipid. The reaction mixture was transferred into a pear-shaped flask, and the solvent was evaporated using a rotary evaporator. To the residue were added DMPC (Nippon Oil and Fats)(17.7 mg), PS (Sigma)(4.0 mg), CH (Wako Pure Chemical Industries)(4.0 mg), and a mixture of chloroform and methanol (9:1 v/v)(5 ml), and they were mixed well to give a solution. The solvent was evaporated under reduced pressure using a rotary evaporator, thereby thin film of lipids was formed on the inner wall of the flask. To the flask was added 10 mM phosphate buffer (3.5 ml), pH 7.2, containing 300 mM sucrose and the air in the flask was replaced by nitrogen gas. The flask was kept at 30°C for one hour to proceed sufficient hydration and the content was stirred for 10 minutes using a vortex mixer to yield a white suspension. The suspension was passed through a polycarbonate membrane filter having a pore diameter of 1 $\mu$m (Nuclepore) ten times, and then through a polycarbonate membrane filter having a pore diameter of 200 nm (Nuclepore) ten times. The filtrate was purified by gel-filtration using a Bio-Gel A 50m column (Bio-Rad) to give liposomes linked to CD4(68-94) peptide.

Turbidity at 540 nm ($OD_{540}$) of the liposomes thus obtained was 0.81, and average vesicle size thereof was 173 nm.

The content of CD4(68-94) peptide linked to the liposomes was measured by reversed phase HPLC under the same conditions as described in Example 10. For this purpose, the liposome sample was first solubilized by addition of octyl glucoside at the final concentration of 50 mM. After addition of dithiothreitol at the final concentration of 50 mM, the mixture was kept at room temperature for 2 hours so that the disulfide bond between phospholipid and CD4(68-94) peptide was be reduced to liberate the thiol group-introduced CD4(68-94) peptide. The reaction mixture (100 $\mu$l) was injected into HPLC, and the amount of the liberated peptide was determined from the peak area corresponding to the peptide. On the other hand, part of the liposome sample was solubilized in the same manner as above and injected into HPLC without reduction by dithiothreitol. This procedure determined the amount of free peptide which had been entrapped within the liposomes without linking thereto.

On the basis of the data as determined in the above, the amount of the peptide linked to the liposomes was calculated by the following equation.

$$\left( \begin{array}{l} \text{Amount of CD4(68-94) peptide} \\ \text{linked to liposomes} \end{array} \right) = \left( \begin{array}{l} \text{Total amount of CD4(68-94)} \\ \text{peptide in liposome solution} \end{array} \right)$$

$$- \left( \begin{array}{l} \text{Amount of free CD4(68-94)} \\ \text{peptide entrapped in liposomes} \end{array} \right)$$

Based on the above equation, the amount of CD4(68-94) peptide linked to the liposomes was 26.1 $\mu$g/ml, or 114 CD4(68-94) peptides per single liposome.

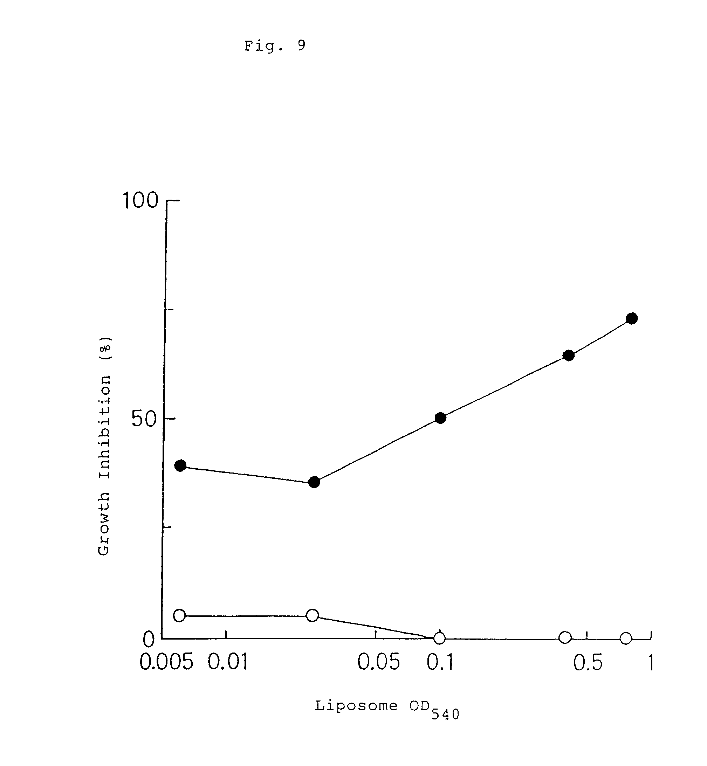

Cytotoxicity of the liposomes of the invention on HIV-infected cells was determined according to the method described in Example 1. The results are shown in Fig. 9. The liposomes showed strong growth inhibition on HIV-infected cells dose-dependently. Concentration ($OD_{540}$) of the liposomes required for inhibiting the growth of the infected cells by 50% was 0.1, which is one seventh concentration of the peptide-free liposomes (Comparative Example 3) necessary for inhibiting the growth in the same way. Thus, the liposomes are effective at very low concentration. On the other hand, they did not show any inhibiting action on the growth of the uninfected cells. Accordingly, it was concluded that the liposomes linked to CD4(68-94) peptide are selectively cytotoxic to HIV-infected cells.

Example 14

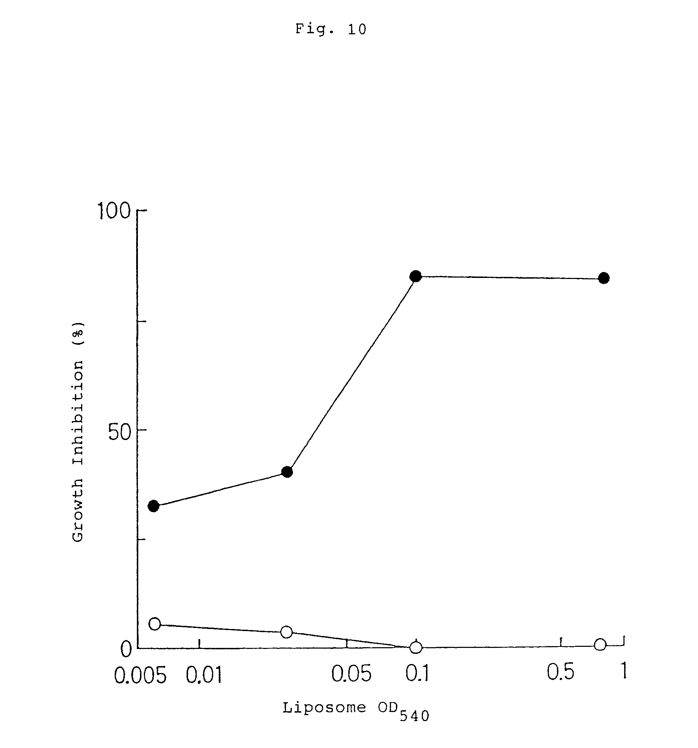

The liposomes linked to CD4(68-94)Bzl peptide were prepared in accordance with the method described in Example 13 except that thiol group-introduced CD4(68-94)Bzl peptide prepared in Example 11 was used in place of thiol group-introduced CD4(68-94) peptide. $OD_{540}$ of the liposomes thus obtained was 0.82, and the average vesicle size was 172 nm. The amount of CD4(68-94)Bzl peptide linked to the liposome was 9.3 $\mu$g/ml, and the number of CD4(68-94)Bzl peptides linked to a single liposome was 41. Cytotoxicity of the liposomes on HIV-infected cells was determined in the same manner as in Example 13. The results are shown in Fig. 10. The liposomes linked to CD4(68-94)Bzl peptide almost completely inhibited the growth of HIV-infected cells at $OD_{540}$ of 0.1 or above. Concentration ($OD_{540}$) of the liposomes required for inhibiting the growth of the infected cells by 50% was 0.04, which is one seventeenth concentration of the peptide-free liposomes (Comparative Example 3) necessary for inhibiting the growth in the same way. On the other hand, the liposomes did not show inhibitory action on the growth of uninfected cells even at the highest concentration. This concludes that the liposomes linked to CD4(68-94)Bzl peptide have strong and selective cytotoxicity to HIV-infected cells.

Example 15

The liposomes linked to CD4(68-94)Acm peptide were prepared in accordance with the method described in Example 13 except that thiol group-introduced CD4(68-94)Acm peptide prepared in Example 12 was used in place of thiol group-introduced CD4(68-94) peptide. $OD_{540}$ of the liposomes thus obtained was 0.94, and the average vesicle size was 176 nm. The amount of CD4(68-94)Acm peptide linked to the liposome was 12.8 $\mu$g/ml, and the number of CD4(68-94)Acm peptides linked to a single liposome was 49.

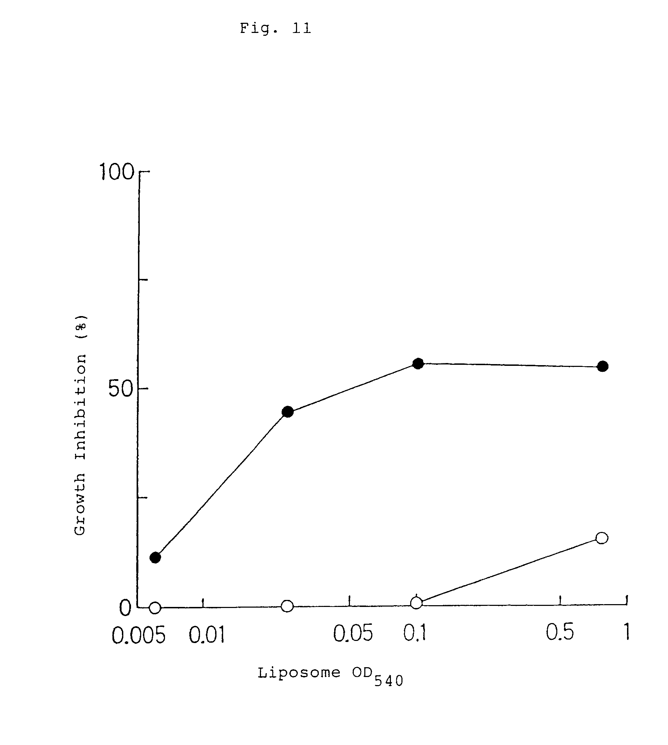

Cytotoxicity of the liposomes on HIV-infected cells was determined in the same manner as in Example 13. The results are shown in Fig. 11. The liposomes linked to CD4(68-94))Acm peptide showed dose-dependent and strong inhibitory action on HIV-infected cells. Concentration ($OD_{540}$) of the liposomes required for inhibiting the growth of the infected cells by 50% was 0.055, which is one twelfth concentration of the peptide-free liposomes (Comparative Example 3) necessary for inhibiting the growth in the same way. On the other hand, the liposomes did not show inhibitory action on the growth of uninfected cells even at the highest concentration. This concludes that the liposomes linked to CD4(68-94)Acm peptide have strong and selective cytotoxicity to HIV-infected cells.

Comparative Example 3

Peptide-free liposomes were prepared according to the method described in Example 13 using as membrane components PDP-PE (3.8 mg), DMPC (35.4 mg), PS (8.0 mg), and CH (8.0 mg), without employing CD4(68-94) or modified CD4(68-94) peptides. Lipids composition of the resultant liposome membrane was DMPC:PS:CH:PDP-PE = 5:1:2:0.4 (molar ratio). $OD_{540}$ of the liposome was 1.73, and the average particle size was 168 nm.

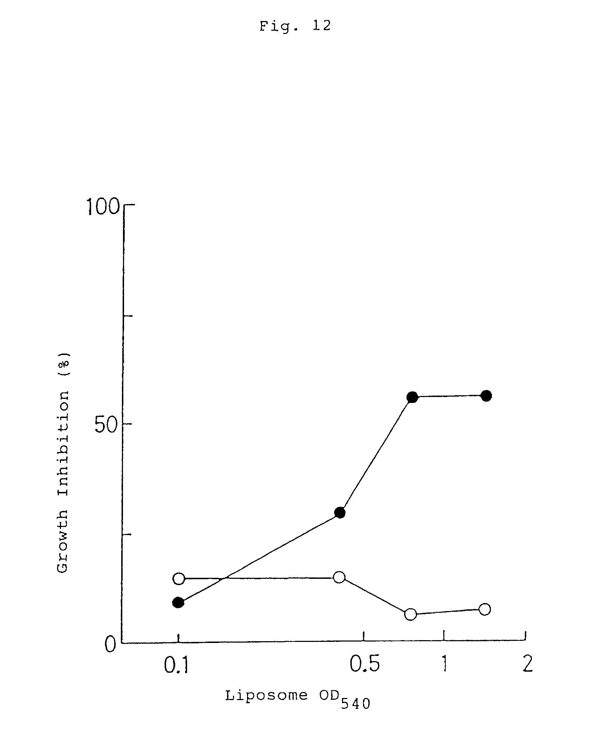

Cytotoxicity of the liposomes on HIV-infected cells was determined in the same manner as in Example 13. The results are shown in Fig. 12. Cytotoxicity to HIV-infected cells was observed at a concentration ($OD_{540}$) of 0.5 or above. Concentration ($OD_{540}$) of the liposomes required for inhibiting the growth of the infected cells by 50% was 0.67. The liposomes did show least inhibitory action on the growth of uninfected cells.

## Claims

1. Liposomes cytotoxic to virus-infected cells which comprise as membrane constituents a phosphatidylcholine having a phase-transition temperature of below 37° C, an acidic phospholipid, and cholesterol.

2. Liposomes of Claim 1, wherein the phosphatidylcholine having a phase-transition temperature of below 37° C is phosphatidylcholine carrying a myristoyl or lauroyl group as an acyl group.

3. Liposomes of claim 1, wherein the phosphatidylcholine having a phase-transition temperature of below 37° C is lecithin of animal origin.

4. Liposomes of any one of Claims 1 to 3, wherein the molar percentages of the phosphatidylcholine, the acidic phospholipid, and the cholesterol are 60-85%, 5-20%, and 10-35%, respectively.

5. Liposomes of Claim 1, wherein the molar ratio of the phosphatidylcholine, the acidic phospholipid, and the cholesterol is 5:1:2.

6. Liposomes cytotoxic to HIV-infected cells which comprise as membrane constituents:
   (a) a phospholipid linked to a peptide possessing anti-HIV activity, said peptide comprising an amino acid sequence:
   Asn-Phe-Pro-Leu-Ile-Ile-Lys-Asn-Leu-Lys-Ile-
   Glu-Asp-Ser-Asp-Thr-Tyr-Ile-Cys-Glu-Val-Glu-
   Asp-Gln-Lys-Glu-Glu
   wherein the Cys residue is L-cysteine, or S-benzylated or S-acetamidomethylated derivative of L-cysteine;
   (b) a phosphatidylcholine having a phase-transition temperature of below 37° C;
   (c) an acidic phospholipid; and
   (d) cholesterol.

7. Liposomes of Claim 6, wherein the phosphatidylcholine is dimyristoylphosphatidylcholine.

8. Liposomes of Claim 6 or 7, wherein the molar percentages of the phospholipid, the phosphatidylcholine, the acidic phospholipid, and the cholesterol are 0.1-10%, 55-85%, 5-20%, and 10-35%, respectively.

9. Liposomes of Claim 6 or 7, wherein the liposome membrane consisting of the phosphatidylcholine, the acidic phospholipid, and the cholesterol at a molar ratio of 5:1:2 contains the phospholipid in an amount of 0.1-10% by mole.

10. Liposomes of any one of Claims 6 to 9, wherein the number of the peptides linked to each liposome is 40-120.

11. The use of the liposomes of any one of Claims 1 to 10 for preparing a medicament for treating virus infections, including HIV- and EBV-infections.

Fig. 1

Fig. 2

Fig. 3

Fig. 4

Fig. 5

Fig. 6

Fig. 7

Fig. 8

Fig. 9

Fig. 10

Fig. 11

Liposome $OD_{540}$

Fig. 12

| European Patent Office | EUROPEAN SEARCH REPORT | Application Number |
|---|---|---|

# DOCUMENTS CONSIDERED TO BE RELEVANT

EP 90123694.3

| Category | Citation of document with indication, where appropriate, of relevant passages | Relevant to claim | CLASSIFICATION OF THE APPLICATION (Int. Cl.5) |
|---|---|---|---|
| Y | WO - A1 - 89/05 633 (LABORATORIUM FÜR EXPERIMEN-TELLE CHIRURGIE) * Abstract; claims 1,2; page 3, third passage; page 4, third passage * | 1,3,4, 6,8,11 | A 61 K 9/127 A 61 K 31/685 A 61 K 37/02 A 61 K 39/00 A 61 K 39/385 A 61 K 47/48 A 61 K 35/14 |
| Y | EP - A2 - 0 179 660 (ELI LILLY AND COMPANY) * Abstract; page 37; example 4; page 9, line 14 - page 11, line 8, page 12, lines 18-24 * | 1,3,4, 6,8,11 | |
| Y | WO - A1 - 89/03 221 (GENELABS, INC.) * Abstract; claims 1-4,10, 11; page 5, lines 7-39; page 8, lines 13-36; page 12, lines 16-24 * | 1,3,4, 6,8,11 | |
| Y | US - A - 4 235 871 (D.P. PAPAHADJOPOULOS) * Claims 1,3,19,22,23; column 2, lines 35-48; column 6, lines 31-43 * | 1,3,4, 6,8,11 | TECHNICAL FIELDS SEARCHED (Int. Cl.5) A 61 K |
| Y | WO - A1 - 88/01 304 (THE TRUSTEES OF COLUMBIA UNIVERSITY) * Abstract; claims 20-27,31, 32; fig. 6A; sequence no. 68-94 * | 1,3,4, 6,8,11 | |
| A | DE - A1 - 3 711 724 (HARRO BOERNER MEDIZINISCHE KOMMUNIKATION GMBH) * Claims 1,4,5 * | 1,3,6, 11 | |
| A | EP - A2 - 0 203 676 | 1,3,6, | |

The present search report has been drawn up for all claims

| Place of search | Date of completion of the search | Examiner |
|---|---|---|
| VIENNA | 22-02-1991 | MAZZUCCO |

EPO FORM 1503 03.82 (P0401)

**European Patent Office**

Application Number

-2-
EP 90123694.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| | (THE WISTAR INSTITUTE OF ANATOMY AND BIOLOGY) <br> * Abstract; page 1, lines 1-24; claim 1 * | 11 | |
| A | EP - A1 - 0 292 000 <br> (SUMITOMO CHEMICAL COMPANY) <br> * Abstract; claims 1-3,6,7; page 3, lines 12-18 * | 1,3-5, 11 | |
| A | WO - A1 - 87/01 592 <br> (YEDA RESEARCH AND DEVELOP-MENT COMP.) <br> * Abstract; claims 1-5; page 5, especially AL 721 * | 1-5,11 | |
| A | EP - A2 - 0 330 227 <br> (THE TRUSTEES OF COLUMBIA UNIVERSITY IN THE CITY OF NEW YORK) <br> * Abstract; claims 1-4 * | 1,3,4, 6,8,11 | |
| A | EP - A2 - 0 335 426 <br> (INSTITUT PASTEUR) <br> * Abstract; claims 1,2 * | 1,3,4, 6,8,11 | TECHNICAL FIELDS SEARCHED (Int. Cl.5) |

The present search report has been drawn up for all claims

| Place of search | Date of completion of the search | Examiner |
|---|---|---|
| VIENNA | 22-02-1991 | MAZZUCCO |

EPO FORM 1503 03.82 (P0401)